# EUROPEAN PATENT SPECIFICATION

(11) **EP 2 671 501 B1**
(45) Date of publication and mention of the grant of the patent: **05.09.2018**
(21) Application number: 13170895.0
(22) Date of filing: 06.06.2013
(51) Int. Cl.: A61B 46/10, A61B 1/00

(54) **A sterile enclosure for an electronic device and a backplate for retaining the electronic device**
Steriles Gehäuse für eine elektronische Vorrichtung und Rückplatte zum Halten der elektronischen Vorrichtung
Enceinte stérile pour dispositif électronique et plaque arrière pour retenir le dispositif électronique

(30) Priority: 07.06.2012 GB 201210063
(43) Date of publication of application: 11.12.2013
(73) Proprietor: Paramount Medical Solutions Ltd, Tunbridge Wells Kent TN4 8ET (GB)
(72) Inventor: Sandor, Rolf, Tunbridge Wells, Kent TN4 8DS (GB)
(74) Representative: Miller Sturt Kenyon

(56) References cited:
- WO-A1-98/02107
- WO-A2-2012/094069
- JP-U- 3 162 334
- TW-U- M 406 911
- US-A- 6 123 080
- US-A1- 2006 247 666
- US-A1- 2010 096 963
- US-A1- 2012 043 235
- US-A1- 2012 074 272
- US-A1- 2012 080 577
- US-B1- 6 845 775
- US-B1- 7 694 814
- US-E- R E33 854

## Description

The present invention relates to a sterile enclosure for an electronic device and in particular, but not exclusively, an electronic device in the form of a portable computer device such as an iPad™. The present invention also relates to a backplate for use in the sterile enclosure.

It is quite common for surgeons during the course of a surgical procedure to wish to monitor the progress of a device, which may have been inserted into the body of a patient. An example of this is a camera, which generates an electronic signal representative of an image of the inside of a patient and outputs the signal for subsequent display. A particularly advantageous and portable display device in use at the present time is the so-called iPad™, which is manufactured by the Apple company. This device can receive externally generated signals and display them in full colour on a relatively large screen. It is therefore possible for such a device to be connected to the output of a camera, image intensifier, or x-ray or ultrasound machine (wirelessly or hardwired), in order to display, enhance or manipulate the image being recorded by these devices. One problem, however, is that the iPad™ is not sterile and therefore care has to be taken with the use of such a device in a sterile field, such as an operating theatre.

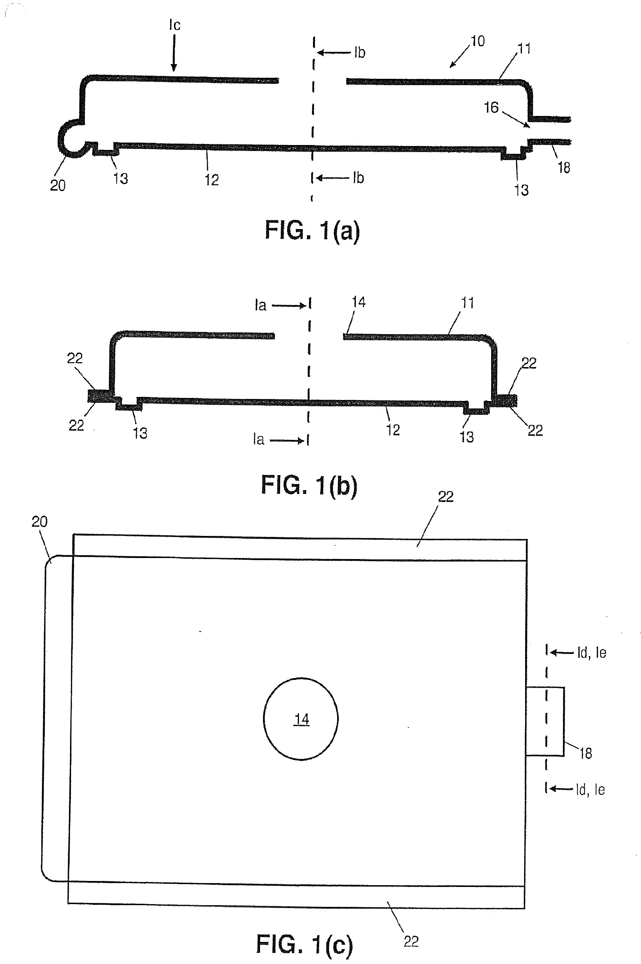

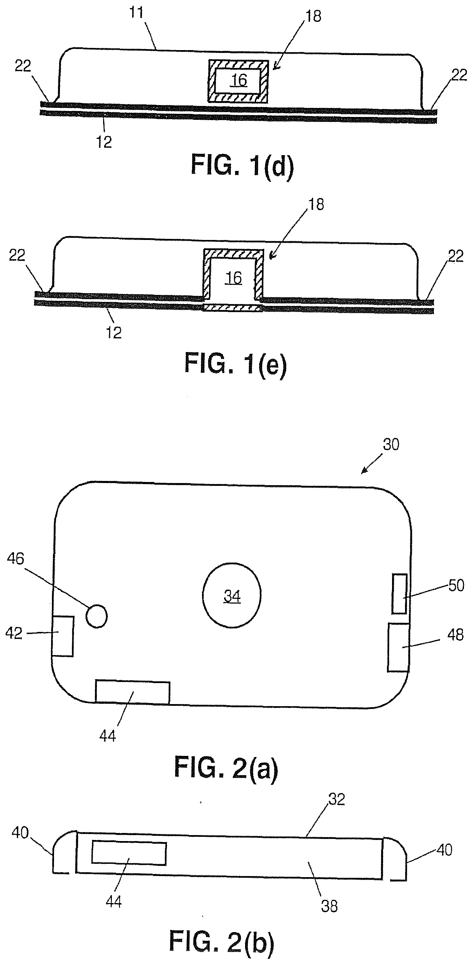

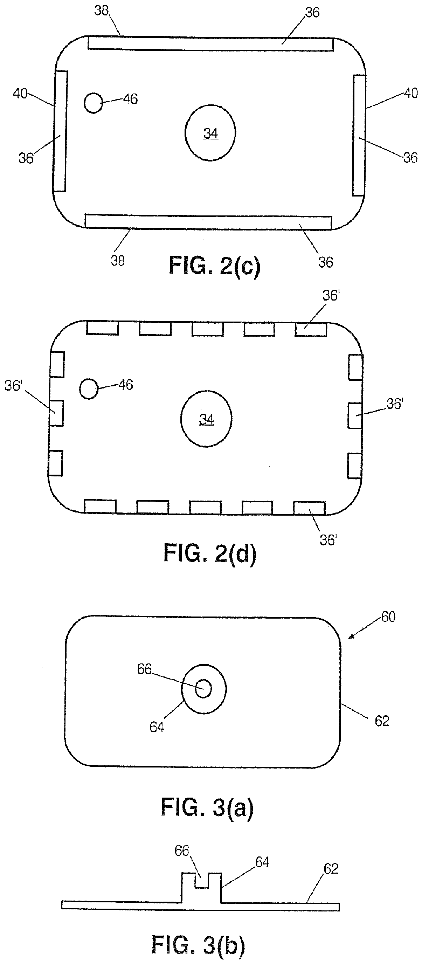

In US 2010/0096963 a surgeon operates a personal electronic device in a sterile environment, in order to control an electronic device in a non-sterile environment (see Fig. 1). Fig. 2 shows a sterile housing for a personal electronic device. The housing comprises a housing made of a material that can be sterilised by, e.g. gas sterilization, dry heat sterilization, radiation, gamma irradiation, etc. The device may be a smart device with, e.g., a touch screen, buttons, etc. and includes a housing that is separate from the sterile housing. The sterile housing includes a cover, a base and a user interface, through which the user can view, touch, access, actuate and control the device. There is also a sterilizable connector for connection to the device. The base has a cavity which accommodates the device. The cover and base are either of the same material or different. The material may be relatively hard or relatively soft or a combination of both. Suitable materials for the housing are: high-temperature thermoplastics, thermoplastic elastomers, thermoset elastomers. Where the cover and base are different, one may be of rubber while the other is of plastic. The user interface includes a protective screen of, e.g., polyethylene, polypropylene, polycarbonate, polymethylpentene, Teflon® or polymethyl methacrylate. This interface is aligned with a user interface of the device, so that the user can operate the device through the interface. A clip assembly may be employed for securing the housing to a surface.

US 6,123,080 relates to a sterile drape used to cover ancillary equipment during a surgical operation. The drape comprises a sleeve, a ring-shaped former and a housing. The sleeve is partially folded over and carried on the former. The former and the folded portion of the sleeve are carried in a recess provided in the housing. A device such as a camera complete with its cables is introduced through a bore and into the sleeve and positioned in a neck portion of the sleeve. A probe for insertion into a patient is then attached to the camera through an aperture formed in the sleeve and the sleeve then sealed around the probe using surgical tape. The sleeve is then unfurled as the housing is moved back along the cables.

In US 6,845,775 a flat video monitor is accommodated inside a sealed chamber. The monitor comprises an LCD screen connected to an electronic module. The chamber comprises a front portion and a rear portion, which is hump-shaped to accommodate the module. The front and rear portions are sealed together by a Teflon® seal. The front and rear portions can both be made of glass or alternatively only the part covering the screen is transparent. An alternative material is plastic, e.g. Makrolon™ DP1-1262 or Noryl™. The chamber is pressurised either positively or negatively relative to its surroundings. The chamber is secured to a surface by an articulated arm. The module has wire connections linked to electrical contacts, which mate with corresponding contacts on the arm.

A sterile enclosure, as described in US 7,694,814, comprises a top section and a bottom section each made from a rigid and thermally and/or chemically stable material, e.g. DERLIN™ or anodized aluminium. The top and bottom sections are connected together by a hinge and secured to each other by, e.g., a bracket, catch, clamp, clasp, clip, latch, hasp, hook, etc. A medical device is inserted into the enclosure. An example of such a device is a scan head assembly comprising an ultrasonic transducer, a cable and a connector. Both of the top and bottom sections have a seal. A cable strain relief is also provided.

US 2012/043235 relates to a protective case for an electronic device which is shock-resistant, chemical-resistant, etc. It is not meant to be sterile. The case is in two halves hinged along one edge. One half accommodates the electronic device and the other half has a screen, through which the user can operate the touchscreen of the device. The two halves are clamped shut by co-operating tabs and protrusions, where the protrusions engage with openings in the tabs. Alternatively, instead of the openings, the tabs may have ramps or claws on their underside, which engage with the protrusions. The two halves may be made watertight by the provision of a seal. An access port is provided to allow at least a portion of a USB cable to be inserted through the port to engage a connector port of the electronic device. The case can be attached to a suitable surface via a base member, which is fixed to the surface, and a bracket, which is swivellably attached to the base member and can be tightened thereto in a desired orientation of the case via a knob.

An anastomosis device is the subject of US 2006/247666. The device includes a tissue approximating structure and an actuating wire attached to an actuating mechanism, which is used for extending and retracting the tissue approximating structure. The actuating mechanism is housed in a protective enclosure. The enclosure comprises first and second body portions joined together by a hinge. An opening at the bottom end of the enclosure accommodates the actuating wire. Closure of the device may be maintained by a locking mechanism - e.g. a clasp or a magnet. The enclosure may be made of a robust material - e.g. metal, rigid or semi-rigid plastic or some other relatively hard material. However, weight is also a factor.

In WO 98/02107 a sterile enclosure accommodates a monitor module, which is used for viewing a surgical area in a sterile operating field. The enclosure includes a flexible body, which can conform to the shape of the monitor module. One end of the enclosure is closed, while the other end is open to receive the monitor module. A seal at the end seals the enclosure after the module has been inserted. The enclosure is made of a transparent and liquid/gas-impermeable material - e.g. polyurethane, polyolefins, polyethylene, polystyrene, laminated plastic films, etc. An integral transparent cover acts as a window allowing the monitor screen to be seen. This cover may be made of, e.g., acrylic or polycarbonate and may be more rigid than the enclosure itself. A cable from the module exits through a cable port and a cable drape seals the cable from the sterile field.

In accordance with a first aspect of the present invention there is provided an enclosure for an electronic device, according to claim 1, comprising a sterile clamshell for housing an electronic device, the clamshell comprising: a first part for receiving the electronic device, a second part whereby the first part can be closed off, and fastening means for retaining the first part in its closed-off state.
The clamshell may be of one-piece construction, the second part of the clamshell being hinged to the first part of the clamshell.
The first and second parts of the clamshell may each have an outwardly protruding flange portion, such that the flange portions abut against each other when the first part of the clamshell is closed off by the second part of the clamshell, the fastening means maintaining the abutted state of the flange portions.
The fastening means may comprise a plurality of hook-and-loop fasteners.

The clamshell comprises an opening, through which a connector can be inserted and mated with a corresponding connector on the electronic device. The opening may communicate with an extension of the clamshell, through which the connector and/or wiring attached to the connector can pass.

A first sterile sleeve is provided for sheathing said extension of the clamshell.

An opening is also provided in a rear wall of the clamshell for receiving an attachment means for attaching the sterile clamshell to a holder. A sterile sleeve is attached to the outside of the rear wall of the clamshell, the sterile sleeve having an sleeve-opening approximately concentric with the opening provided in the rear wall of the clamshell. The sterile sleeve may be a telescopic sleeve, which is adjustable between a fully retracted state and an extended state.
The sterile clamshell may comprise a material selected from a group consisting of PET, PE and PVC.

In accordance with a another aspect of the present invention, there is provided a backplate for holding an electronic device, the backplate comprising a plate having three terminal points and, at said terminal points, respective retaining members for retaining opposite edges of the electronic device.
The plate may be triangular in shape. The triangle may be complete or with one side missing.
The plate may have a central portion disposed intermediate the three terminal points.

The retaining members at two of the three terminal points may have an L-shaped profile for holding two of the corners of the electronic device. The remaining retaining member may be configured as a flexible clip, with which an edge of the electronic device opposite to the edge common to the two corners, can be brought into engagement. The backplate may comprise an attachment member attached to the central portion on a side of the plate opposite to the side which is to face the electronic device.
The backplate may comprise a thermally conductive element disposed on the side of the triangular plate which is to face the electronic device. The plate may comprise a material of high thermal conductivity.

In accordance with another aspect of the present invention, there is provided a sterile enclosure for an electronic device, comprising: the sterile clamshell as described above; a backplate with retaining means for retaining the electronic device, and an attachment means for attaching the sterile enclosure to a holder; the clamshell being adapted to receive the backplate with the electronic device in its retained state.
At least the second part of the clamshell may be transparent and the electronic device may be a portable computer device having a display screen, the display screen being viewable through the transparent second part of the clamshell. The display screen may be a touch-sensitive display screen which can be operated through the clamshell. Advantageously, the backplate of the sterile enclosure is a backplate having the features described above.
Embodiments of the invention will now be described, by way of example only, with reference to the drawings, of which:
Figs. 1(a) and 1(b) are cross-sectional views of a clamshell used in an embodiment of a sterile enclosure according to the present invention, while Fig. 1(c) is a plan view of the clamshell and Figs. 1(d) and 1(e) are cross-sectional views of an extension portion of the clamshell;
Figs. 2(a)-2(c) are a plan view, a side view and an underside view, respectively, of a backshell used in an embodiment of a sterile enclosure according to the present invention; while Fig. 2(d) is a variant of the view shown in Fig. 2(c);
Figs. 3(a) and 3(b) are a plan view and a side view, respectively, of a heatsink plate used in an embodiment of a sterile enclosure according to the present invention;
Fig. 4 is a sectional view of a sterile enclosure according to the present invention and incorporating the clamshell, backshell and heatsink plate shown in Figs. 1-3;
Fig. 5 shows a fastener used to clamp together the two parts of the clamshell;
Fig. 6 is a first embodiment of a flexible mounting device for mounting a sterile enclosure according to the present invention to a fixed point;
Fig. 7 is a second embodiment of a flexible mounting device for mounting a sterile enclosure according to the present invention to a fixed point;
Fig. 8 is a side view of a telescopic sterile sleeve, which may be used as part of the sterile enclosure of Fig. 4;
Fig. 9 is an alternative version of a backshell used in an embodiment of a sterile enclosure according to the present invention;
Figs. 10(a) and 10(b) are side views of a backshell without and with the use of a separate heatsink plate, respectively;
Figs. 11(a) and 11(b) show the use of a backplate, rather than a backshell, and involve two different examples of a retaining means;
Fig. 12 is a plan view of a further embodiment of a backplate with an electronic device installed on it;
Figs. 13 and 14 are exploded views of a specific embodiment of the backplate shown in Fig. 12, and
Fig. 15 is a side view of the backplate assembly of Figs. 13 and 14.

An embodiment of a sterile enclosure in accordance with the present invention comprises a clamshell 10 as shown in Figs. 1(a)-1(c). Figs. 1(a) and 1(b) are two orthogonal cross-sectional views of the clamshell intended to receive a device such as an iPad™. The iPad™ is longer than it is wide and this is reflected in the aspect-ratio of the clamshell. Thus, the length of the clamshell, as shown in Fig. 1(a) is greater than its width, as shown in Fig. 1(b). The clamshell is of one-piece construction and comprises a first part 11, which receives the iPad™, and a second part 12, which closes off the opening at the front of the first part, thereby to form a sterile enclosure extending all around the iPad™ except at a substantially central location at the back of the first part 11, where an opening 14 is provided, and a location in one side of the first part, where an opening 16 is provided. The opening 16 communicates with an extension 18 of the first part 11, the purpose of which will become apparent later. The first and second parts 11, 12 of the clamshell are joined at the end of the clamshell opposite the opening 16 by a hinge 20. Extending all around at least two opposite sides of each of the first and second parts 11, 12 of the clamshell is a flange 22. These flanges butt up against each other when the second part of the clamshell is in its fully closed position, as shown in Figs. 1(a) and 1(b). Once in its fully closed position, a fixing means (not shown in Figs. 1(a) and 1(b)) is used to clamp the flanges together. Recesses 13 are provided in the second part of the clamshell for a reason which will become apparent later.

A cross-sectional view of the extension 18 is shown in two different versions in Figs. 1(d) and 1(e). These drawings show the first part 11 and the second part 12 of the clamshell butted up to each other. Also visible are the flanges 22. The extension 18 is rectangular in both cases, but in Fig. 1(d) it is formed entirely in the first part 11, while in Fig. 1(e) it is formed partly in the first part 11 and partly in the second part 12. Also, instead of being rectangular, the extension 18 and opening 16 may be oval or circular in shape, or any other profile which allows a connector to be inserted into the iPad™.

Figs. 2(a)-2(c) show a plan view, a side view and an underside view, respectively, of a backshell 30, which is used in conjunction with the clamshell 10.

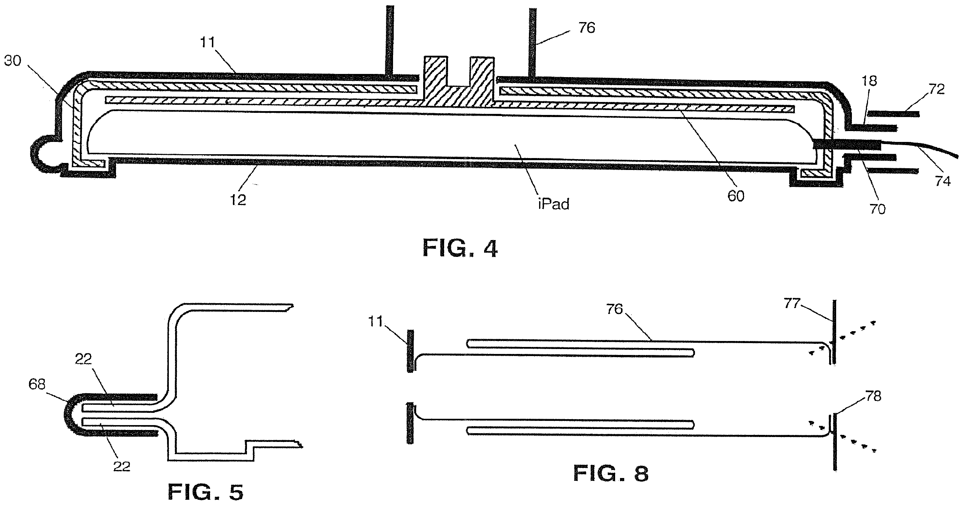

The backshell is of unitary construction and is shaped so as to be able to receive the iPad™, which is to be placed in the clamshell. Like the clamshell, the backshell has in its rear wall 32 an opening 34, which corresponds to the opening 14 in the clamshell. Also provided are lips 36, which serve to retain the iPad™ in the backshell. The lips 36 are right-angle extensions of respective side-walls 38, 40 of the backshell. The side-walls are not continuous around the periphery of the backshell, but are separate items. This allows the side-walls to flex slightly as the iPad™ is inserted into the backshell past the lips 36. The backshell is meant to be a fairly rigid item and therefore some difficulty may be experienced in inserting the iPad™ into it. In view of this, a variant of the backshell is shown in Fig. 2(d), in which the lips 36 are not continuous along each edge, but are divided into a plurality of smaller lips 36'. This provides less resistance to the insertion of the iPad™, while at the same time providing a reliable retention action, once the iPad™ is in place. More or fewer small lips 36' may be provided than are shown in Figs. 2(d) and their widths may vary.
As is well known, the iPad™ has a number of operating elements for use by the user. These include a sleep/wake button, a volume control switch, a mute switch, a camera and a docking connector. A speaker is also provided. The backshell 30 is therefore provided with openings corresponding to the locations of these operating elements. These openings are an opening 42 for the sleep/wake button, an opening 44 for the volume and mute switches, an opening 46 for the camera and an opening 50 for the docking connector. An opening 48 may also be provided for the speaker. The opening 46 is provided in the rear wall of the backshell, while the openings 42, 44 and 50 are provided in respective side-walls.
Turning now to Figs. 3(a) and 3(b), these show a heatsink plate 60, which is used in conjunction with the backshell 30.
The heatsink plate is formed of a suitably heat-conductive material, e.g. aluminium, and comprises a flat plate portion 62 and a boss portion 64. The boss portion, which constitutes an attachment means, has a female thread 66 for mating with a male thread on a connection member, which will be described later.
The whole arrangement is assembled as will now be described with reference to Fig. 4.

Firstly, the heatsink plate 60 is inserted into the backplate 30 so that the boss portion 64 protrudes through the opening 34 in the backshell. Then the iPad™ is inserted into the backshell so that it is retained by the lips 36 or 36' and the backshell with iPad™ is inserted into the clamshell 10 so that the boss portion 64 protrudes through the opening 14 formed in the clamshell, and the second portion 12 of the clamshell is then closed shut. During this process the iPad™ is oriented so that its screen is visible to the user. At that point the first and second parts 11, 12 of the clamshell are clamped together by the fixing of a series of sterile hook-and-loop fasteners 68 across the abutting flanges 22 of the clamshell (see Fig. 5). Thus, the fasteners 68 will have the hooks, while the flanges will have the loops, or vice-versa. These fasteners are preferably substantially equidistantly spaced along each side of the clamshell. Preferably they are employed along the long sides of the clamshell, but may also be employed along the short side at the end opposite the hinge 20. Once the clamshell is closed shut, a connector 70 is inserted through the extension 18 and opening 16 in the clamshell and through the opening 50 in the backshell, so that the iPad™ can be connected to suitable apparatus (e.g. a camera) in the operating theatre in which the iPad™ is to be used after being placed into the backshell. A sterile sleeve 72 may be used to sheath the clamshell extension 80 and at least part of the lead 74 coming from the connector 70.

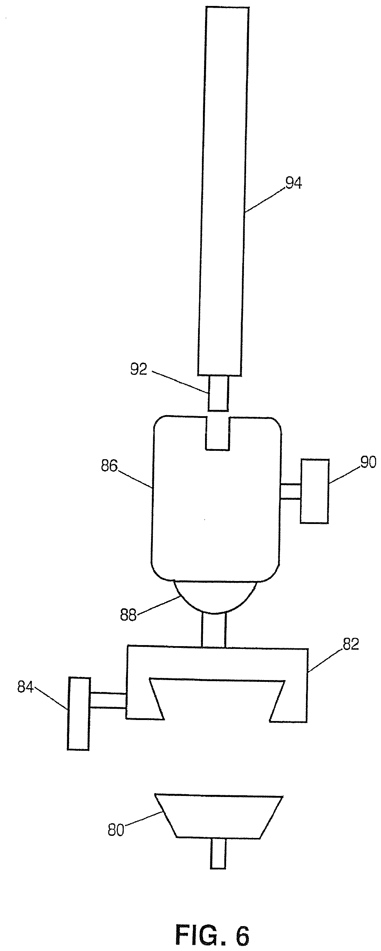

At this point the sterile enclosure may be attached to a fixed point in the operating theatre. This is achieved in one embodiment of the present invention by attaching a connection member to the boss portion 64 of the heatsink plate. Such a connection member is shown in Fig. 6 as item 80. Item 80 is the male part of a quick-disconnect coupling, the other part being the female part 82. The connection member 80 is wedge-shaped and slides into a correspondingly shaped opening in the female part 82. Once in place, the connection member 80 can be secured in place by the operation of a screw 84.

In the arrangement shown in Fig. 6 the female part 82 is connected to a universal joint mechanism 86 comprising a joint ball 88. The movement of the joint ball can be frozen by the operation of a screw 90. Finally, the universal joint mechanism 86 is attached by a threaded bolt 92 to one end of a flexible arm 94, the other end being anchored to a convenient fixed point in the operating theatre. A sterile sleeve 76 (see Fig. 4) is fitted over part or all of the flexible mounting device shown in Fig. 6.

This arrangement allows the user to orient the iPad™ into any orientation by adjustment of the universal joint mechanism and the flexible arm. This is essential for a surgeon, who requires complete freedom to operate and for whom, therefore, the iPad™ needs to be in exactly the right place.

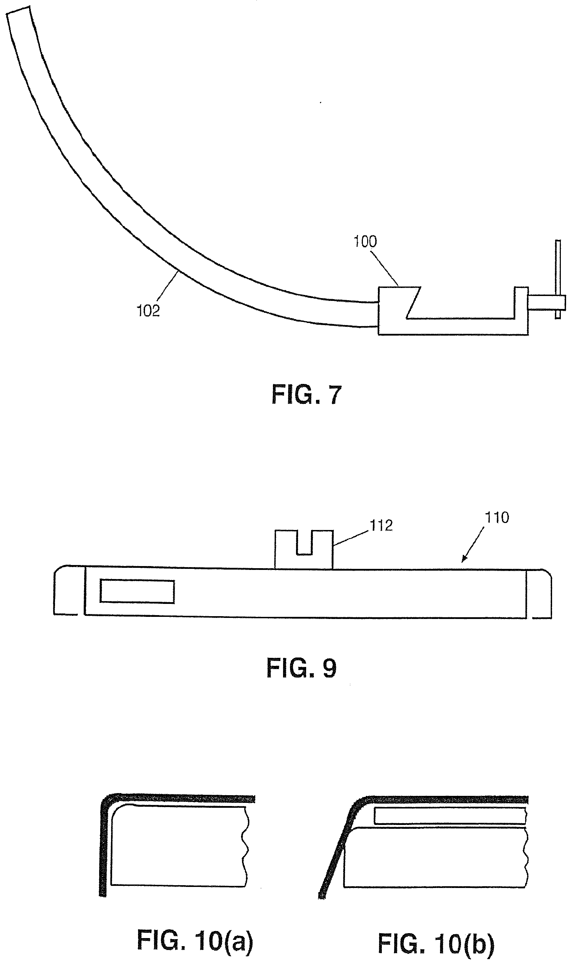

An alternative mounting arrangement is shown in Fig. 7. In Fig. 7 the connection member 80 is again slid into a female part. This time, however, the female part takes the form of a so-called funnel frame 100, also sometimes called a "surgi-funnel". The funnel frame is directly attached to a flexible arm 102, which corresponds to the flexible arm 94 in Fig. 6. Thus in this alternative arrangement the universal joint mechanism has been omitted, resulting in a simpler configuration. The degree of freedom of the iPad™ is more restricted in Fig. 7, but this may be acceptable under some circumstances.

As regards the sterile sleeve 76 (see Fig. 4), this is preferably a telescopic-type sleeve having two states: a fully retracted state, in which the sleeve is telescoped together, so as to assume its minimum length, and an extended state, in which the sleeve is pulled out to either its maximum or some intermediate extent. It is also ready-attached to the back of the clamshell, e.g. by an adhesive or some other suitable fixing means, so that it does not have to be fitted separately.

In practice, when the backshell with the heatsink plate, boss and iPad™ is inserted into the clamshell, the boss will be received in the telescoped sleeve 76. At that point the clamshell can be closed up and the quick-release connector 80 (see Fig. 6) screwed to the boss. The whole enclosure can then be offered up to the mounting arrangement of Fig. 6 or Fig. 7, i.e. the boss is mated to either the female coupling part 82 or the surgi-funnel 100, and the sleeve 76 is then pulled out so that it covers preferably the whole of the flexible arm 94 or 102, all the way to the wall or desk, to which the arm is mounted.

An example of a telescopic sleeve is illustrated in Fig. 8. The sleeve, which is made of a very flexible material, e.g. PE, is attached at its proximal end to the first part 11 of the clamshell, as already mentioned, and at its distal end to an extension member 77. The attachment at both ends is made with the aid of an adhesive. The extension member 77 is made of card, or any suitable stiff material, and is approximately circular in shape, looking along the longitudinal axis of the sleeve 76, though it may have any other suitable shape, e.g. rectangular. It has a circular hole 78 in its central portion. In use, the sleeve starts off folded up (telescoped) onto the rear side of the extension member 77, which lies approximately parallel to the first part 11 of the clamshell (see the solid line 77). It is assumed that the boss already protrudes through the opening 78 of the extension member 77 and that the male part 80 of the quick-disconnect connector is attached to the boss. When the clamshell is to be secured to the mounting arrangement shown in Fig. 6 or Fig. 7, the male part 80 is coupled to the female part 82 or 100, the opposite ends of the extension member 77 are pinched between the finger and thumb, so that the extension member assumes the dotted-line configuration in Fig. 8, and the distal end of the sleeve is then pulled over the mounting arrangement. The extension member is then released.

Although it has been assumed that a separate heatsink plate will be employed, in practice the role of backshell and heatsink plate may be combined into one component. Fig. 9 shows such an arrangement, in which a backshell 110 is formed from a heat-conductive material (e.g. aluminium) and is itself provided with a boss portion 112. In this case it is essential that substantially the whole of the rear side of the iPad™ be in contact with the rear wall of the backshell, so that heat can be efficiently conducted away from the iPad™. This may require that the sidewalls of the backshell be substantially orthogonal to the rear wall of the backshell, as shown in Fig. 10(a). By contrast, when a separate heatsink plate is used, the sidewalls may come off at an angle to the rear wall (see Fig. 10(b)).

The iPad™ will normally be used as a display or as a remote-control for an image scanning device, in which case the clamshell assembly will be fitted to the mounting arrangement of Fig. 6 or Fig. 7 so that the screen of the iPad™ faces the surgeon. The surgeon may also wish to record a surgical procedure, instead of viewing it. In that case, the assembly needs to be oriented so that the camera hole 46 in the backshell faces the surgeon or operating site. This can be done using the mounting arrangements of Fig. 6 or Fig. 7.

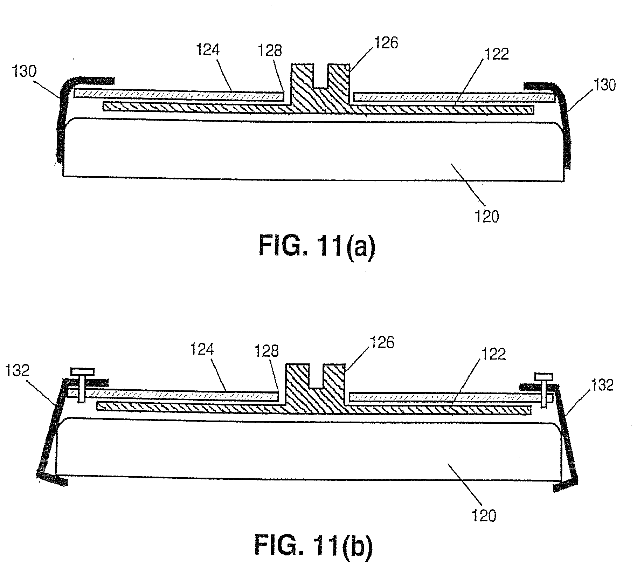

Instead of using a backshell, as described above, it is possible to use a backplate, to which the electronic device is fixed by some suitable means - e.g. using hook-and-loop fasteners similar to that shown in Fig. 5. An example of such an arrangement is shown in Fig. 11(a), in which an iPad™ 120 is in contact with a heat-sink plate 122, which in turn is in contact with a backplate 124. The heat-sink plate 122 has a central boss 126, which passes through a hole 128 provided in the backplate. Holding the iPad™ in place against the backplate is a series of hook-and-loop fasteners 130, of which only two are shown. The hook and loop fasteners may be provided spaced apart over two or preferably four edges of the backplate in order to provide maximum retention. It should be noted that the hook and loop fasteners need not extend around the sides of the iPad™ but may simply be provided as pads attached to the back of the iPad™ and the front of the backshell/back plate/heatsink.

An alternative form of fixing is illustrated in Fig. 11(b). Here the hook-and-loop fasteners are replaced by spring clips, one end of which is secured to a peripheral portion of the backplate 124, while the other end is in contact with the peripheral portion of the front face of the iPad™. The spring clips 132 will normally be oriented more vertically than shown, but will be splayed out as the iPad™ is offered up to the heat-sink plate, assuming the position shown in Fig. 11(b).

It should also be noted that the backshell need not be provided with side walls on all of its sides. If the iPad™ can be expected to be used in only one or two orientations, it is sufficient to provide a wall that will support only the bottom edge of the iPad™ in that orientation. Thus, the backshell/backplate may comprise a back wall and one supporting wall arranged to abut a side wall of the iPad™ in use.

Although the boss 64 and the male part 80 have been shown as having female and male threads, respectively, these may be reversed.

The extension 18 of the clamshell has been described as having four sides. However, this is not strictly necessary, provided the connector 70 (see Fig. 4) is sufficiently shrouded. Thus, the extension may only have three sides (e.g. the side in the second portion 12 of the clamshell in Fig. 1(e) is omitted), or perhaps only two. In the latter case it is preferable that the sides of the extension parallel with the top and bottom of the connector be present and somewhat wider than the connector. Moreover, it is envisaged that the extension 18 may not be present, but may be replaced with a telescopic sleeve similar to the sleeve 76. In that case, the sleeve may be similarly ready-attached to the clamshell. The connector can then be inserted through the fully retracted sleeve and through the opening 16 and mated with the iPad's docking connector. The sleeve can then be pulled out to cover the connector 70 and at least part of the wire attached to the connector 70.

In what has been described above, the clamshell is used to provide a sterile enclosure to the iPad™. It may therefore be made of any suitable non-rigid material, which is transparent and does not tear easily. In addition, the material must be such as to allow the user to operate the iPad™ properly. This involves the need to operate the touch screen of the iPad™, e.g. by tapping, swiping, drawing finger and thumb together or apart for zooming purposes, etc.. This may require that the second part 12 especially of the clamshell lie substantially flat against the iPad™ screen. To assist in this, recesses 13 have been provided in the second part 12 of the clamshell shown in Figs. 1(a) and 1(b). These recesses accommodate the lips 36, 36' of the backshell, as shown in Fig. 4, allowing the inner face of the second part 12 of the clamshell to abut against the display of the iPad™. In addition, the material used for the clamshell must be flexible enough to allow the user to operate the operating elements mentioned in connection with the backshell (see Fig. 2). Transparency allows the user to view the iPad™ screen and for the camera of the iPad™ to record a scene. Suitable materials meeting all of these requirements are PET (polyethylene terephlalate), PE (polyethylene) and PVC (polyvinylchloride), though the invention is not limited to these.

Providing the necessary rigidity for the sterile enclosure is the backshell, which may be used either on its own, in which case it will preferably have a heatsinking property, or together with a separate heatsink plate. Suitable materials for the backshell are a ceramic material, aluminium or some other suitable alloy.

It has so far been assumed that the clamshell will be of one-piece construction. However, an alternative is to use a two-piece construction, in which case provision will be made for the second part of the clamshell to at least lightly clip onto the first part, proper securing then being achieved through the use of the afore-mentioned hook-and-loop fasteners. Also, it is not absolutely necessary for the two parts of the clamshell to have the flanges 22 shown in Figs. 1(a) and 1(b). Instead, these may be omitted and the hook-and-loop fasteners instead secured to, e.g. the side-walls of the first part 11 of the clamshell and to the front face of the second part 12. Similarly, even when flanges are used, the hook-and-loop fasteners may be secured in this manner, rather than to the flanges themselves.

Where a one-piece clamshell is employed, the hinge joining the two parts of the clamshell may be situated either as shown in Fig. 1(c), or along one of the long sides. In that case, where the flanges 22 are included, one of them will be on the short side of the clamshell, since the hinge is now on a long side.

Although the heatsinking arrangement has taken the form of either a backshell with heatsinking properties or a separate heatsink used with a non-heatsinking backshell, in practice both a heatsinking backshell and a separate heatsink plate may be employed. This allows the backshell to be thin enough to allow the side wall to flex, (this being necessary for an iPad™ to be inserted into the backshell), while at the same time increasing the thickness of the heatsinking material at the interface between the iPad and the rear wall of the clamshell.

Since the backplate/backshell, heat sink and boss are enclosed within the sterile clamshell and sleeve 76, it is not necessary for them to be sterilised before inserting them into the clamshell.

What has been described is a sterile enclosure for an electronic device, such as an iPad™, which allows the electronic device to be used in an unhindered manner. Thus, the clamshell is such that it allows the user to operate the electronic device easily and reliably. Hence, a device such as an iPad™ can not only be used as a display for the user (e.g. a surgeon) to monitor what is being recorded by, e.g., a camera or x-ray device. It can also be used to remotely control a device, which would normally require a separate operator. Hence a surgeon during a procedure can call up a patient's notes or previous scans by operating the iPad™ through the clamshell. This dispenses with the need for a separate computer for this purpose. Assisting in this function is the use of a suitable material, which will not distort during use and will therefore allow accurate operation of, e.g., the icons displayed on an iPad™ touch screen. In addition, the provision of a quick-disconnect type connector enables the user to easily hook the sterile enclosure, complete with electronic device, up to a fixing member, which is attached to a fixed point in the sterile field. Indeed, there may be many such fixing members distributed around the sterile field. By employing a flexible arm (optionally with a universal joint) with the connector, the user can orient the sterile enclosure so that it is within his easy reach, allowing him to operate a camera, x-ray machine, etc., or access relevant notes or scans, without having to walk over to a non-sterile computer. Thus the device removes unwanted distance between the user and the equipment he may wish to monitor or control with the sterile enclosure.

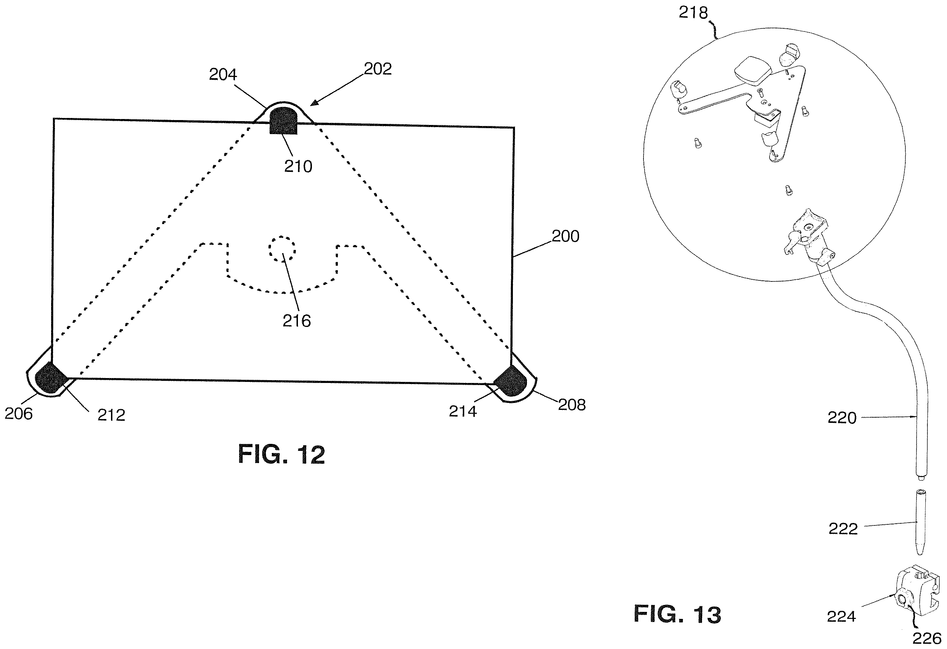

An alternative form of backplate is illustrated in plan view in Fig. 12. The drawing shows an iPad™-type device 200 mounted on a backplate 202. The backplate is generally triangular in shape, but with one side missing, having terminal points 204, 206 and 208 on which are attached respective retaining members 210, 212, 214, which hold the iPad™ in place roughly midway along one edge and at its opposite two corners. A hole 216 approximately at the centre of the backplate is used to attach the backplate to a mount, which in turn is attached to a clamp on a wall or other surface.

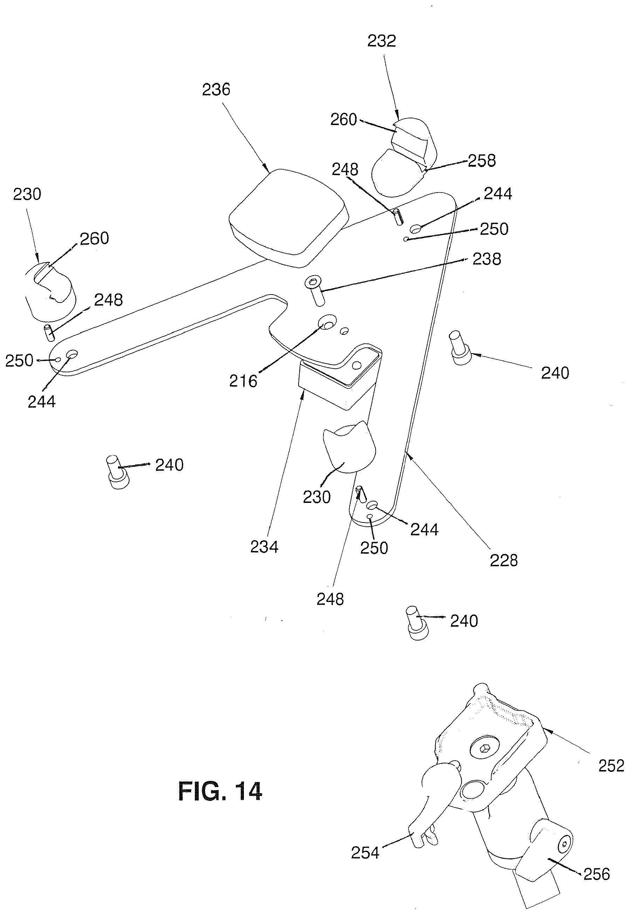

A detailed embodiment of the backplate of Fig. 12 will now be described with reference to Figs. 13 and 14.

Fig. 13 shows the backplate and mount assembly 218, a flexible support arm 220, a straight clamp insert 222 and a clamp assembly 224. The support arm 220 slots into the clamp insert 222, which in turn slots into the clamp assembly 224, being tightened in place by a screw knob 226.

Fig. 14 is an enlarged version of the inset 218 in Fig. 13. The backplate assembly comprises the backplate proper 228, a pair of corner retaining members 230, a hinge retaining member 232, a carrier-mount plate 234 and a thermally conductive silicone pad 236. The carrier-mount plate 234 is held in place underneath the backplate 228 by a set screw 238, while the retaining members 230, 232 are held in place by bolts 240. To prevent unwanted rotation of the retaining members 230, 232, a pin 248 is inserted through a hole 250 at each terminal point and into a corresponding hole formed in the retaining members.

The carrier-mount plate 234 engages with a standard tripod-type carrier mount 252. The mount 252 has a spring-loaded latch 254, which is urged against a side of the plate 234 and retains it in place inside the mount 252. The other end of the mount 252 has a lever 256, which is used to tighten against the top end of the flexible support arm 220, thereby preventing the mount 252 from rotating against the support arm 220. The lever 256, however, allows the iPad™ to be oriented as desired, e.g. in either portrait or landscape orientation.

In use, the iPad™ is fitted over the silicone pad 236 and its corners brought into engagement with the corner retaining members 230, which have an L-shaped engaging surface (i.e. L-shaped in the plane of the backplate) for this purpose. The top edge of the iPad™ is then clipped into the hinge retaining member 232. This clipping action is facilitated by the provision of a narrow bridge section 258 on the retaining member 232, which allows the outer section of this retaining member to bend backwards as the top edge of the iPad™ is moved down onto the retaining member 232. The three retaining members have a V-shaped groove 260 (i.e. V-shaped in a direction perpendicular to the plane of the backplate), with which the relevant parts of the iPad™ can engage.

The silicone pad 236 acts as a heat-transfer mechanism to pass some of the heat generated in the iPad™ on to the backplate 228. The backplate is therefore preferably made of a material having high conductivity, e.g. aluminium. However, an alternative material is stainless steel, preferably with a Teflon™ coating, an anodised finish or a powder coating. The retaining members may be made of polypropylene, for example, or nylon.

Although the triangular backplate has been described as having the orientation shown, in practice it may be turned upside down, so that the hinge retaining member 232 is at the bottom and the two corner retaining members are at the top. This is not the preferred orientation, however. This is because, depending on the orientation of the iPad™, there may be a substantial pull downwards onto the hinge retaining member, due to gravity. This, and the fact that the bridge section 258 is quite weak, relatively speaking, could make the iPad™ less secure on the backplate, compared to the illustrated situation, in which the corner retaining members 230 are at the bottom.

Instead of using corner retaining members, like the retaining members 230 in Fig. 14, it is possible to use retaining members similar to the retaining member 232, but without the bridge section 258. This would enable an electronic device wider than the iPad™ to be accommodated on the same backplate, but could have the drawback that the device could end up sliding off the backplate, depending on its orientation. This would especially be a problem if the iPad™ were orientated in portrait mode. Mainly, however, it is envisaged that the backplate will be custom-made for the particular electronic device in question, so that corner retaining members will generally be used. Manufacturing a custom-made backplate is quite easy in practice, since it can be laser-cut to any particular size, and similarly the retaining members can be machined to be a suitable size for any device.

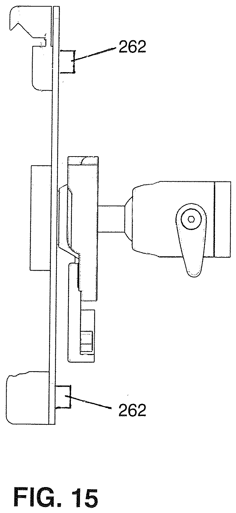

As regards fitting the assembled backplate assembly complete with iPad™, this backplate assembly simply replaces the backplate 30 in Fig. 4. Consequently, the backplate assembly with iPad™ is inserted into the first part 11 of the clamshell with the carrier-mount plate 234 protruding from the opening in that first part 11. In a preferred embodiment of the clamshell for the Figs. 12-14 embodiment of the backplate, there will be three recesses in the first part 11 for receiving the heads of the bolts 240, which protrude on the underside of the backplate. This is more clearly seen in the side view of Fig. 15, which shows two of the bolt-heads 262. The provision of such recesses in the clamshell has the advantage that it becomes even clearer to the user which way round the backplate needs to be inserted into the clamshell.

Although the backplate has been illustrated and described as being in the form of an incomplete triangle, in practice it may be a complete triangle, i.e. having all three sides. Furthermore, the central section having the mounting hole 216 may be integral with only part of the plate, as shown in Fig. 12, or may be integral with the whole of the rest of the plate. Such a backplate would therefore have a continuous plate area (except for the mounting hole 216) between all three terminal points.

Fig. 12 shows the backplate being configured to accept an iPad™ in its landscape orientation. Alternatively, the backplate may be configured to accept the iPad™ in its portrait orientation. This, of course, would mean ensuring that the distance between the corner retaining members 212, 214 was less than the distance between the corner retaining members and the remaining retaining member 210. As a further alternative, the backplate could be configured to accept a substantially square-profile electronic device.
It has already been mentioned that the hole 216 in Fig. 12 will be in roughly the centre of the backplate. In a preferred embodiment of the backplate, this hole will lie at the expected centre of mass of the electronic device being accommodated. As regards the location of the heat-transfer pad 236, this is preferably located adjacent to a part of the electronic device which produces the most heat.
Although the embodiment of Figs. 13 and 14 has assumed the use of a straight insert 222 linking the flexible support arm 220 with the clamp assembly 224, the straight insert may be dispensed with and the flexible arm directly coupled to the clamp assembly. The foregoing description has been given by way of example only and it will be appreciated by a person skilled in the art that modifications can be made without departing from the scope of the appended claims.

## Claims

1. A sterile enclosure for an electronic device, comprising:
a sterile clamshell (10) for housing an electronic device;
a backplate (30) with retaining means (36, 36', 130, 132, 210, 212, 214) for retaining the electronic device, and
an attachment means (64, 112, 126, 234) for attaching the sterile enclosure to a holder (94, 102, 220);
wherein the sterile clamshell (10) comprises:
a first part (11) for receiving the electronic device;
a second part (12) whereby the first part (11) can be closed off;
fastening means (68) for retaining the first part (11) in its closed-off state;
an opening (14) provided in a rear wall of the clamshell (10), the opening (14) for receiving the attachment means (64, 112, 126, 234); and
a sterile sleeve (76) attached to the outside of the rear wall of the clamshell (10), the sterile sleeve (76) having a sleeve-opening approximately concentric with the opening (14) provided in the rear wall of the clamshell (10), the sterile sleeve (76) being adjustable between a retracted state and an extended state;
wherein the backplate (30) is received in the sterile clamshell (10), the attachment means (64, 112, 126, 234) protruding through the opening (14) provided in the rear wall of the clamshell (10) and being surrounded by the sterile sleeve (76).

2. The sterile enclosure according to claim 1, wherein the sterile sleeve (76) is retracted such as to allow the attachment means (64, 112, 126, 234) to be attached to the holder (94, 102, 220).

3. The sterile enclosure according to claim 1, wherein the attachment means (64, 112, 126, 234) is attached to the holder (94, 102, 220) and the sterile sleeve (76) is extended over all or part of the holder (94, 102, 220).

4. The sterile enclosure according to any one of the preceding claims, wherein
the clamshell (10) is of one-piece construction, the second part (12) of the clamshell being hinged to the first part (11) of the clamshell (10).

5. The sterile enclosure according to any one of the preceding claims, wherein the first and second parts (11, 12) of the clamshell (10) each have an outwardly protruding flange portion (22), such that the flange portions (22) abut against each other when the first part (11) of the clamshell (10) is closed off by the second part (12) of the clamshell (10), the fastening means (68) maintaining the abutted state of the flange portions (22).

6. The sterile enclosure according to any one of the preceding claims, wherein the clamshell (10) comprises a connector opening (16), through which a connector (70) can be inserted and mated with a corresponding connector on the electronic device.

7. The sterile enclosure according to claim 6, wherein said connector opening (16) of the clamshell (10) communicates with an extension (18) of the clamshell (10), through which the connector (70) and/or wiring (74) attached to the connector (70) can pass.

8. The sterile enclosure according to claim 7, comprising a further sterile sleeve (72) for sheathing said extension (18) of the clamshell (10).

9. The sterile enclosure according to any one of the preceding claims, wherein the sterile clamshell (10) comprises a material selected from a group consisting of PET, PE and PVC.

10. The sterile enclosure according to any one of the preceding claims, wherein at least the second part (12) of the clamshell (10) is transparent and the electronic device is a portable computer device having a display screen, the display screen being viewable through the transparent second part (12) of the clamshell (10).

11. The sterile enclosure according to claim 10, wherein the display screen is a touch-sensitive display screen which can be operated through the clamshell (10).

12. The sterile enclosure according to any one of the preceding claims, wherein:
the attachment means (64, 112, 126, 234) is a part of the backplate (30).

13. The sterile enclosure according to any one of claims 1 to 11, wherein the attachment means (64) is part of a heatsink (60, 62, 122) provided between the backplate (30, 124) and the electronic device.

14. The sterile enclosure according to any one of the preceding claims, wherein the backplate (30) comprises:
a plate (202) having three terminal points (204, 206, 208); and
at said terminal points (204, 206, 208), respective retaining members (210, 212, 214) for retaining opposite edges of the electronic device.

15. The sterile enclosure according to claim 14, wherein:
the plate (202) is triangular in shape.

16. The sterile enclosure according to claim 14, wherein:
the plate (202) is triangular in shape, but with one side of the triangle missing,

## Patentansprüche

1. Steriles Gehäuse für eine elektronische Vorrichtung, umfassend:
ein steriles Klappgehäuse (10) zum Unterbringen einer elektronischen Vorrichtung;
eine Rückplatte (30) mit Haltemitteln (36, 36', 130, 132, 210, 212, 214) zum Halten der elektronischen Vorrichtung, und
ein Befestigungsmittel (64, 112, 126, 234) zum Befestigen des sterilen Gehäuses an einer Halterung (94, 102, 220);
wobei das sterile Klappgehäuse (10) umfasst:
ein erstes Teil (11) zur Aufnahme der elektronischen Vorrichtung;
ein zweites Teil (12), wodurch das erste Teil (11) verschlossen werden kann;
Befestigungsmittel (68) zum Halten des ersten Teils (11) in seinem verschlossenen Zustand;
eine Öffnung (14), die in einer Rückwand des Klappgehäuses (10) vorgesehen ist, wobei die Öffnung (14) zur Aufnahme der Befestigungsmittel (64, 112, 126, 234) dient; und
eine sterile Hülse (76), die an der Außenseite der Rückwand des Klappgehäuses (10) befestigt ist, wobei die sterile Hülse (76) eine Hülsenöffnung aufweist, die annähernd konzentrisch zu der in der Rückwand des Klappgehäuses (10) vorgesehenen Öffnung (14) ist,
wobei die sterile Hülse (76) zwischen einem zurückgezogenen Zustand und einem ausgestreckten Zustand einstellbar ist;
wobei die Rückplatte (30) in dem sterilen Klappgehäuse (10) aufgenommen ist, wobei die Befestigungsmittel (64, 112, 126, 234) durch die in der Rückwand des Klappgehäuses (10) vorgesehene Öffnung (14) hervorstehen und von der sterilen Hülse (76) umgeben sind.

2. Steriles Gehäuse nach Anspruch 1, wobei die sterile Hülse (76) derart zurückgezogen ist, dass ermöglicht wird, die Befestigungsmittel (64, 112, 126, 234) an der Halterung (94, 102, 220) zu befestigen.

3. Steriles Gehäuse nach Anspruch 1, wobei das Befestigungsmittel (64, 112, 126, 234) an der Halterung (94, 102, 220) befestigt ist und die sterile Hülse (76) über die gesamte oder einen Teil der Halterung (94, 102, 220) ausgedehnt ist.

4. Steriles Gehäuse nach einem der vorhergehenden Ansprüche, wobei das Klappgehäuse (10) eine einteilige Konstruktion ist, wobei das zweite Teil (12) des Klappgehäuses an das erste Teil (11) des Klappgehäuses (10) angelenkt ist.

5. Steriles Gehäuse nach einem der vorhergehenden Ansprüche, wobei das erste und das zweite Teil (11, 12) des Klappgehäuses (10) jeweils einen nach außen vorstehenden Flanschabschnitt (22) derart aufweisen, dass die Flanschabschnitte (22) aneinander anliegen, wenn das erste Teil (11) des Klappgehäuses (10) durch das zweite Teil (12) des Klappgehäuses (10) verschlossen ist, wobei die Befestigungsmittel (68) den anliegenden Zustand der Flanschabschnitte (22) aufrechthalten.

6. Steriles Gehäuse nach einem der vorhergehenden Ansprüche, wobei das Klappgehäuse (10) eine Anschlussöffnung (16) aufweist, durch die ein Anschlussteil (70) eingeführt und mit einem entsprechenden Anschlussteil an dem elektronischen Gerät verbunden werden kann.

7. Steriles Gehäuse nach Anspruch 6, wobei die Anschlussöffnung (16) des Klappgehäuses (10) mit einem Verlängerungsstück (18) des Klappgehäuses (10) in Verbindung steht, durch welches das Anschlussteil (70) und/oder die an dem Anschlussteil (70) befestigte Verdrahtung (74) hindurchgehen kann.

8. Steriles Gehäuse nach Anspruch 7, umfassend eine weitere sterile Hülse (72) zum Umhüllen des Verlängerungsstücks (18) des Klappgehäuses (10).

9. Steriles Gehäuse nach einem der vorhergehenden Ansprüche, wobei das sterile Klappgehäuse (10) ein Material umfasst, das ausgewählt ist aus einer Gruppe, bestehend aus PET, PE und PVC.

10. Steriles Gehäuse nach einem der vorhergehenden Ansprüche, wobei mindestens das zweite Teil (12) des Klappgehäuses (10) transparent ist und die elektronische Vorrichtung eine tragbare Computervorrichtung mit einem Bildschirm ist, wobei der Bildschirm durch das transparente zweite Teil (12) des Klappgehäuses (10) sichtbar ist.

11. Steriles Gehäuse nach Anspruch 10, wobei der Bildschirm ein berührungsempfindlicher Bildschirm ist, der durch das Klappgehäuse (10) bedient werden kann.

12. Steriles Gehäuse nach einem der vorhergehenden Ansprüche, wobei:
das Befestigungsmittel (64, 112, 126, 234) ein Teil der Rückplatte (30) ist.

13. Steriles Gehäuse nach einem der Ansprüche 1 bis 11, wobei das Befestigungsmittel (64) Teil eines Kühlkörpers (60, 62, 122) ist, der zwischen der Rückplatte (30, 124) und der elektronischen Vorrichtung vorgesehen ist.

14. Steriles Gehäuse nach einem der vorhergehenden Ansprüche, wobei die Rückplatte (30) umfasst:
eine Platte (202), die drei Endpunkte (204, 206, 208) aufweist; und
an den Endpunkten (204, 206, 208) entsprechende Halteelemente (210, 212, 214) zum Halten von gegenüberliegenden Kanten der elektronischen Vorrichtung.

15. Steriles Gehäuse nach Anspruch 14, wobei:
die Platte (202) eine dreieckige Form aufweist.

16. Steriles Gehäuse nach Anspruch 14, wobei:
die Platte (202) eine dreieckige Form aufweist, aber eine Seite des Dreiecks fehlt.

## Revendications

1. Enceinte stérile pour dispositif électronique, comprenant :
une coque stérile (10) pour loger un dispositif électronique ;
une plaque arrière (30) avec des moyens de retenue (36, 36', 130, 132, 210, 212, 214) pour retenir le dispositif électronique, et
des moyens de fixation (64, 112, 126, 234) pour fixer l'enceinte stérile à un support (94, 102, 220) ;
dans laquelle la coque stérile (10) comprend :
une première partie (11) pour recevoir le dispositif électronique ;
une seconde partie (12) par laquelle la première partie (11) peut être fermée ;
un moyen de fixation (68) pour retenir la première partie (11) dans son état fermé ;
une ouverture (14) prévue dans une paroi arrière de la coque (10), l'ouverture (14) pour recevoir les moyens de fixation (64, 112, 126, 234) ; et
un manchon stérile (76) fixé sur l'extérieur de la paroi arrière de la coque (10), le manchon stérile (76) ayant une ouverture de manchon approximativement concentrique avec l'ouverture (14) prévue dans la paroi arrière de la coque (10),
le manchon stérile (76) étant ajustable entre un état rétracté et un état étendu ;
dans laquelle la plaque arrière (30) est reçue dans la coque stérile (10), les moyens de fixation (64, 112, 126, 234) faisant saillie à travers l'ouverture (14) prévue dans la paroi arrière de la coque (10) et étant entourés par le manchon stérile (76).

2. Enceinte stérile selon la revendication 1, dans laquelle le manchon stérile (76) est rétracté de façon à permettre aux moyens de fixation (64, 112, 126, 234) d'être fixés au support (94, 102, 220).

3. Enceinte stérile selon la revendication 1, dans laquelle les moyens de fixation (64, 112, 126, 234) sont fixés au support (94, 102, 220) et le manchon stérile (76) est étendu sur tout ou partie du support (94, 102, 220).

4. Enceinte stérile selon l'une quelconque des revendications précédentes, dans laquelle la coque (10) est de construction monobloc, la seconde partie (12) de la coque étant articulée sur la première partie (11) de la coque (10).

5. Enceinte stérile selon l'une quelconque des revendications précédentes, dans laquelle les première et seconde parties (11, 12) de la coque (10) ont chacune une partie de bride (22) en saillie vers l'extérieur, de telle sorte que les parties de bride (22) butent l'une contre l'autre lorsque la première partie (11) de la coque (10) et fermée par la seconde partie (12) de la coque (10), le moyen de fixation (68) maintenant l'état en butée des parties de bride (22) .

6. Enceinte stérile selon l'une quelconque des revendications précédentes, dans laquelle la coque (10) comprend une ouverture de connecteur (16) à travers laquelle un connecteur (70) peut être inséré et mis en correspondance avec un connecteur correspondant sur le dispositif électronique.

7. Enceinte stérile selon la revendication 6, dans laquelle ladite ouverture de connecteur (16) de la coque (10) communique avec une extension (18) de la coque (10) à travers laquelle le connecteur (70) et/ou le câblage (74) fixé au connecteur (70) peu(ven)t passer.

8. Enceinte stérile selon la revendication 7, comprenant un manchon stérile supplémentaire (72) pour gainer ladite extension (18) de la coque (10).

9. Enceinte stérile selon l'une quelconque des revendications précédentes, dans laquelle la coque (10) stérile comprend un matériau sélectionné parmi un groupe consistant en le PET, le PE et le PVC.

10. Enceinte stérile selon l'une quelconque des revendications précédentes, dans laquelle au moins la seconde partie (12) de la coque (10) est transparente et le dispositif électronique est un dispositif informatique portable ayant un écran d'affichage, l'écran d'affichage étant visible à travers la seconde partie (12) transparente de la coque (10).

11. Enceinte stérile selon la revendication 10, dans laquelle l'écran d'affichage est un écran d'affichage tactile qui peut être actionné à travers la coque (10) .

12. Enceinte stérile selon l'une quelconque des revendications précédentes, dans laquelle :
les moyens de fixation (64, 112, 126, 234) font partie de la plaque arrière (30).

13. Enceinte stérile selon l'une quelconque des revendications 1 à 11, dans laquelle le moyen de fixation (64) fait partie d'un dissipateur thermique (60, 62, 122) prévu entre la plaque arrière (30, 124) et le dispositif électronique.

14. Enceinte stérile selon l'une quelconque des revendications précédentes, dans laquelle la plaque arrière (30) comprend :
une plaque (202) ayant trois points terminaux (204, 206, 208) ; et
sur lesdits points terminaux (204, 206, 208), des membres de retenue (210, 212, 214) respectifs pour retenir des bords opposés du dispositif électronique.

15. Enceinte stérile selon la revendication 14, dans laquelle :
la plaque (202) est de forme triangulaire.

16. Enceinte stérile selon la revendication 14, dans laquelle :
la plaque (202) est de forme triangulaire mais avec un côté du triangle manquant.
